# EUROPÄISCHE PATENTSCHRIFT

(11) **EP 0 923 739 B1**
(45) Veröffentlichungstag und Bekanntmachung des Hinweises auf die Patenterteilung: **14.11.2001**
(21) Anmeldenummer: 97932742.6
(22) Anmeldetag: 05.07.1997
(51) Int. Cl.: G01N 33/574

(54) **VERFAHREN ZUM NACHWEIS EINER HORMON- BZW. ANTIHORMONRESISTENZ BEI TUMOREN**
METHOD FOR DETECTING A HORMONE OR ANTIHORMONE RESISTANCE IN CANCERS
PROCEDE POUR DECELER UNE RESISTANCE AUX HORMONES ET/OU ANTIHORMONES DE TUMEURS

(30) Priorität: 12.07.1996 DE 19628298
(43) Veröffentlichungstag der Anmeldung: 23.06.1999
(73) Patentinhaber: MAX-DELBRÜCK-CENTRUM FÜR MOLEKULARE MEDIZIN, 13125 Berlin (DE)
(72) Erfinder: NAUNDORF, Helga, D-32657 Lemgo (DE); NEUMANN, Claudia, D-16321 Bernau (DE); FICHTNER, Iduna, D-13125 Berlin (DE); BECKER, Michael, D-13187 Berlin (DE)
(74) Vertreter: Baumbach, Friedrich, Dr.
(86) Internationale Anmeldenummer: DE9701423
(87) Internationale Veröffentlichungsnummer: WO9802747

(56) Entgegenhaltungen:
- EP-A- 0 129 669
- US-A- 4 742 000
- US-A- 5 292 638
- US-A- 5 384 260
- HEIKKI ET AL: "Immunocytochemical detection of estrogen and progesterone receptors in 124 human breast cancers" AMJ.CLIN.PATHOL, Bd. 90, Nr. 2, Juli 1988, Seite 137-142 XP002045395

## Beschreibung

Die Erfindung betrifft ein Verfahren zum Nachweis einer Hormon- bzw. Antihormonresistenz bei Tumoren. Anwendungsgebiete der Erfindung sind die Medizin, die Biologie und die pharmazeutische Industrie.

Tumoren in hormonabhängigen Organen, wie Brust und Ovarien, bilden die häufigsten Geschwulsterkrankungen bei Frauen in Industrieländern. So wird die Diagnose Brustkrebs jährlich bei etwa 12 % der weiblichen Bevölkerung gestellt, wobei der Erkrankungsgipfel zwischen dem 4. und 6. Lebensjahrzehnt liegt; 3,5 % der Frauen sterben daran (Harris et. al., New England J Med 327: 319-328, 1992).

Die Heilungschancen bei Brustkrebs liegen je nach Stadium zum Zeitpunkt der Diagnose zwischen 30 und 70 %. Da etwa 2/3 der gefundenen Tumoren über Hormonrezeptoren verfügen, werden bei diesen Patienten nach chirurgischer Entfernung des Primärtumors Hormonpräparate als Erstmedikation eingesetzt. Diese sollen kompetitiv den natürlichen Liganden (Östrogen) vom Rezeptor verdrängen und damit das hormonell bedingte Wachstum der Tumoren beeinflussen. Tamoxifen, ein nichtsteroidales Antiöstrogen, wird zur Behandlung vom Mammacarzinomen am häufigsten eingesetzt, wobei der adjuvante Gebrauch zur Vermeidung der Metastasen-entstehung aber auch die prophylaktische Anwendung bei Patienten mit familiär bedingtem Risiko im Vordergrund stehen (Harris et. al., New England J Med 327: 473-480, 1992).

Es ist bekannt, daß von den hormonrezeptorpositiven Patienten nur etwa die Hälfte auf eine Tamoxifentherapie ansprechen. Dies wirft Fragen zum prinzipiellen Wirkmechanismus der Antiöstrogene auf, die derzeit weitestgehend ungeklärt sind. So beschrieben Berthois et. al. (Molecular and Cellular Endrocrinology 99: 259-268, 1994), daß die Immunreaktivität von östrogenrezeptoren gegenüber einem Antikörper erhöht wird, wenn in einem Cytosoltest Östradiol oder Tamoxifen zugesetzt werden. Die Autoren diskutieren als möglichen Mechanismus für die scheinbare Zunahme der Epitopbindungsstellen eine Konformationsänderung des Östrogenrezeptors durch die in vitro Interaktion mit Hormonen und Antihormonen.

Obwohl Tamoxifen auch im Langzeitgebrauch relativ gut verträglich ist, wird in letzter Zeit sein mögliches kanzerogenes Potential diskutiert (Williams et. al., Eur J Cancer Prev 1: 386-387, 1992).

Die Erfindung hat das Ziel, den Einsatz von Antihormonen zur Tumortherapie dahingehend zu verbessern, daß überflüssige Therapien möglichst vermieden werden. Ihr liegt die Aufgabe zugrunde, ein Verfahren zu entwickeln, mit dem vor einer entsprechenden Antihormontherapie das Ansprechen eines Tumors vorhergesagt werden kann.

Diese Aufgabe wird mit Hilfe des Verfahrens gelöst, das durch Anspruch 1 und die Unteransprüche gekennzeichnet ist. Ausgangspunkt der Erfindung ist der überraschende Befund, daß sich die Immunreaktivität von sensitiven und von resistenten Tumoren unter bestimmten Reaktionsbedingungen unterscheidet. Werden im Tumor vorhandene Hormonrezeptoren an einen Antikörper gebunden, dann wird ihre Immunreaktivität im Falle sensibler Tumoren durch Zugabe der entsprechenden Hormone oder Antihormone erhöht, im Falle resistenter Tumore dagegen nicht. Es findet also nur bei sensiblen Tumoren eine scheinbare Zunahme der Epitopbindungsstellen statt.

Dieser Effekt wird zur Konzipierung des erfindungsgemäßen Verfahrens genutzt. Es ist dadurch gekennzeichnet, daß ein an eine feste Phase gebundener Antihormonrezeptor-Antikörper mit einem aus dem zu untersuchenden Tumorgewebe präparierten Cytosol inkubiert wird, nachfolgend die Flüssigkeit entfernt und unter Zusatz der entsprechenden Hormone bzw. Antihormone erneut inkubiert wird und anschließend ein zweiter enzymmarkierter Antikörper zugesetzt, die Farbreaktion gemessen und der ermittelte Wert für die Immunreaktion mit dem in gleicher Weise, jedoch ohne Inkubation mit den entsprechenden Hormonen oder Antihormonen, ermittelten Kontrollwert verglichen wird.

Unter Hormonen werden in dieser Beschreibung östrogene (wie 17β-Östradiol), Progesterone oder Androgene verstanden, unter Antihormonen Antiöstrogene (wie Tamoxifen oder 4-Hydroxytamoxifen), Antiprogestine oder Antiandrogene (wie Cyproteronacetat).

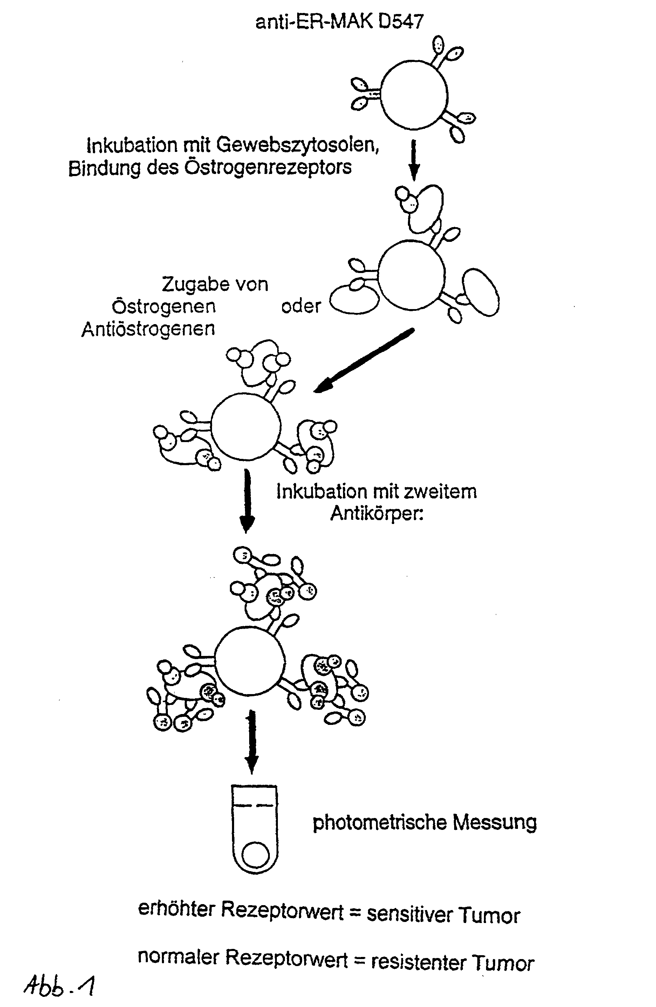

Das Verfahren wird im einzelnen anhand des Beispiels östrogen beschrieben. Man nutzt einen handelsüblichen Enzymimmunoassay, der in der klinischen Routine zur Bestimmung von Hormonrezeptoren eingesetzt wird. Polystyrolkügelchen, die mit einem Anti-Östrogenrezeptor-Antikörper bedeckt sind, werden mit Cytosol, das aus dem entsprechenden Tumorgewebe präpariert wurde, inkubiert. Nach Bindung der vorhandenen Östrogenrezeptoren an den Primär-Antikörper wird die Flüssigkeit entfernt und unter Zusatz von Östrogen bzw. Antiöstrogenen wie Tamoxifen erneut inkubiert. Anschließend wird ein zweiter, Peroxydase-gekoppelter Antikörper zugesetzt und dessen Farbreaktion gemessen, was eine direkte Kalkulation des vorhandenen Östrogenrezeptors gestattet (Abbildung 1).

Die Erfindung ermöglicht eine zuverlässige Unterscheidung von sensitiven und resistenten Tumoren und damit eine Abschätzung der Erfolgschancen einer antitumoralen Hormontherapie.

Die Erfindung soll nachfolgend durch ein Ausführungsbeispiel näher erläutert werden.

### Beispiel

Die Resultate beruhen auf Experimenten mit einem Hormonrezeptor-positiven Mammacarzinom (3366), das aus Patientenmaterial als Xenotransplantationsmodell auf nude Mäuse etabliert wurde (H. Naundorf et. al., J. Cancer Res Clin Oncol 119: 35-40, 1992). Von diesem ursprünglich äußerst Tamoxifen-sensitiven Tumor wurde durch mehrjährige in vivo Behandlung mit suboptimalen Dosen eine resistente Sublinie erzeugt.

Die zu untersuchenden Tumore des Mammakarzinoms 3366 werden nach Entnahme schockgefroren und bis zur Testdurchführung in flüssigem Stickstoff aufbewahrt. Die Homogenisierung der Proben erfolgt mittels Ultraschall bei 2 - 8 °C im Eisbad 5 mal (10 sec.).

Cytosole von der Tamoxifen-sensitiven und der Tamoxifenresistenten Linie werden im von der Firma Abbott entwickelten Enzymimmunoassay eingesetzt. Dieser Enzymimmunoassay enthält den Anti-Östrogen-Rezeptor-Antikörper D547 und als 2. Antikörper den Peroxidase-gekoppelten Antikörper H222, dessen Farbreaktion nach Zugabe von o-Phenyldiamin bei 492 nm gemessen wird. Das Cytosol wird entsprechend der Vorschrift des Abbott ER-EIA-Monoclonal-Kit aufbereitet und der Proteingehalt auf 1 - 2 mg/ml eingestellt.
Nach erfolgter Inkubation mit den Antikörperkügelchen (D 547) wird die Flüssigkeit mit 8 - 12 ml destilliertem Wasser mittels eines Qwikwash-Gerätes (Abbott) abgesaugt. Zu den Kügelchen werden je 200 µl des entsprechenden Hormons bzw. Antihormons in Doppelbestimmung pipettiert. Es wurden 4-Hydroxytamoxifen, 17β-Estradiol, ICI 182,780 (Wakeling, J. Steroid biochem Molec Biol 47: 107-114, 1991) und Progesteron in Konzentrationen von 10⁻⁴ bis 10⁻¹⁰ molar verwendet. Die optimalsten Ergebnisse erzielt man mit 10⁻⁶ molaren, wäßrigen Lösungen, die aus einer 10⁻⁴ molaren ethanolischen Lösung hergestellt werden. In der Kontrollprobe wird nur das Lösungsmittel ohne Hormonzusatz verwendet.
Die Verweildauer der Hormone bzw. Antihormone an den Kügelchen beträgt 2 Stunden, die Temperatur 37 °C (Brutschrank).
Danach erfolgt erneut das Absaugen der Flüssigkeiten mit dem Qwikwash und Zugabe des zweiten Antikörpers H222. Nach Reaktion mit der o-Phenyldiamin-Salzsäurelösung wird entsprechend Abbott-Vorschrift die Messung der ER-Rezeptoren in fmol/ml bzw. der Extinktion bei 492 nm vorgenommen.

Die Ergebnisse sind in nachfolgender Tabelle zusammengefaßt:

Immunreaktivität (in Prozent) gegenüber dem Antikörper H222:

| Behandlung | Mammacarcinom 3366 | |
|---|---|---|
| | Tamoxifen-sensitiv | Tamoxifen-resistent |
| ohne | 100 | 100 |
| 4-Hydroxytamoxifen | 262 ± 53⁺ | 140 ± 13 |
| 17β-Östradiol | 243 ± 45⁺ | 116 ± 17 |
| ICI 182,780 | 291 ± 100⁺ | 123 ± 5 |
| Progesteron | 91 ± 8 | 93 ± 10 |

| | | |
|---|---|---|
| (⁺ signifikant im Vergleich zur resistenten Linie) | | |

Mit den dargestellten Resultaten wird belegt, daß eine signifikante Erhöhung der Immunreaktivität nur in (Anti-) Hormon-sensitiven Tumoren stattfindet, während bei resistenten Tumoren keine zusätzlichen Antikörperbindungsstellen geschaffen werden. Damit läßt sich dieses Testverfahren zur Unterscheidung von Hormon- und Antihormon-sensitiven und - resistenten Tumoren einsetzen.

## Patentansprüche

1. Verfahren zum Nachweis einer Hormon- bzw. Antihormonresistenz, **dadurch gekennzeichnet, daß** man die Immunreaktivität von Tumoren mißt, indem ein an eine feste Phase gebundener Antihormonrezeptor-Antikörper mit einem aus dem zu untersuchenden Tumorgewebe präparierten Cytosol inkubiert wird, nachfolgend die Flüssigkeit entfernt und unter Zusatz entsprechender Hormone bzw. Antihormone erneut inkubiert wird, anschließend ein zweiter enzymmarkierter Antikörper zugesetzt, die Farbreaktion gemessen und der ermittelte Wert für die Immunreaktion mit dem in gleicher Weise, jedoch ohne Inkubation mit den entsprechenden Hormonen oder Antihormonen, ermittelten Kontrollwert verglichen wird.

2. Verfahren nach Anspruch 1, **dadurch gekennzeichnet, daß** ein an eine feste Phase gebundener Antiöstrogenrezeptor-Antikörper mit einem aus dem zu untersuchenden Tumorgewebe präparierten Cytosol inkubiert wird, nachfolgend die Flüssigkeit entfernt und unter Zusatz von Östrogen bzw. von Tamoxifen oder anderer Antiöstrogene erneut inkubiert wird, anschließend ein zweiter Peroxidase-gekoppelter Antikörper zugesetzt, die Farbreaktion gemessen und der ermittelte Wert für die Immunreaktion mit dem in gleicher Weise ermittelten Kontrollwert verglichen wird.

3. Verfahren nach Anspruch 1 oder 2, **dadurch gekennzeichnet, daß** die Inkubation mit einer 10⁻⁵ bis 10⁻⁷ molaren Lösung der entsprechenden Hormone bzw. Antihormone erfolgt.

## Claims

1. Method for the detection of a hormone or anti-hormone resistance, wherein the immune reactivity of tumours is measured by an anti-hormone receptor antibody bound to a solid phase being incubated with a cytosol prepared from the tumour tissue to be examined, the liquid subsequently being removed and being incubated again with the addition of corresponding hormones or anti-hormones, after which a second enzyme-marked antibody is added, the colour reaction being measured and the determined figure for the immune reaction being compared with the control figure determined in the same way, albeit without incubation with the corresponding hormones or anti-hormones.

2. Method according to Claim 1, wherein an anti-oestrogen receptor antibody bound to a solid phase is incubated with a cytosol prepared from the tumour tissue to be examined, the liquid is subsequently removed and incubated again with oestrogen or tamoxifen or other anti-oestrogens, after which a second peroxidase-coupled antibody is added, the colour reaction is measured and the figure determined for the immune reaction is compared with the control figure determined in the same way.

3. Method according to Claims 1 or 2, wherein the incubation is done with a 10⁻⁵ to 10⁻⁷ molar solution of the corresponding hormones or anti-hormones, as the case may be.

## Revendications

1. Procédé permettant la mise en évidence d'une résistance aux hormones, plus précisément anti-hormonale se caractérisant par le fait de mesurer la réactivité immunitaire de tumeurs en faisant incuber un anticorps - récepteur anti-hormone fixé en phase solide avec un cytosol préparé à partir du tissu tumoral à examiner, en éliminant ensuite le liquide et en faisant incuber une nouvelle fois ayant ajouté des hormones resp. des anti-hormones, en ajoutant ensuite un deuxième anticorps marqué aux enzymes, en mesurant la réaction modifiant la couleur et en comparant la valeur déterminée pour la réaction immunitaire avec la valeur de référence déterminée de la même manière, cependant sans incubation avec les hormones ou anti-hormones correspondants.

2. Procédé selon la revendication 1, se caractérisant par le fait de faire incuber un anticorps - récepteur anti-oestrogène fixé en phase solide avec un cytosol préparé à partir du tissu tumoral à examiner, d'éliminer ensuite le liquide et de faire incuber une nouvelle fois ayant ajouté des oestrogènes resp. du Tamoxifène ou d'autres antioestrogènes, d'ajouter ensuite un deuxième anticorps combiné à une peroxydase, de mesurer la réaction modifiant la couleur et de comparer la valeur déterminée pour la réaction immunitaire avec la valeur de référence déterminée de la même manière.

3. Procédé selon la revendication 1 ou 2, se caractérisant par le fait que l'incubation est réalisée avec une solution à 10⁻⁵ à 10⁻⁷ moles des hormones resp. anti-hormones correspondants.
